# EUROPEAN PATENT SPECIFICATION

(11) **EP 0 898 982 B1**
(45) Date of publication and mention of the grant of the patent: **15.09.2004**
(21) Application number: 98305154.1
(22) Date of filing: 29.06.1998
(51) Int. Cl.: A61N 2/06

(54) **Magnetotherapy device**
Einrichtung zur Magnetotherapie
Dispositif de magnétothérapie

(30) Priority: 02.07.1997 GB 9713906
(43) Date of publication of application: 03.03.1999
(73) Proprietor: Ecoflow Limited, Saltash, Cornwall PL12 6TR (GB)
(72) Inventor: Broderick, Nigel, Saltash, Cornwall PL12 6TR (GB)
(74) Representative: Craske, Stephen Allan

(56) References cited:
- EP-A- 0 774 275
- WO-A-82/03177
- BE-A- 700 056
- DE-U- 9 413 430
- US-A- 5 226 020

## Description

### TECHNICAL FIELD OF THE INVENTION

This invention relates to a device for use in magnotherapy as practised on the human or animal body.

### BACKGROUND

Magnotherapy is a therapeutic process which is sometimes performed by physiotherapists using a pulsed magnetic field to alleviate certain diseases and generally improve the health of the recipient. However, the equipment used is large and extremely expensive and would not be practical for use on a personal basis.

It is also known to simulate the pulsing of a magnetic field using fixed magnets placed adjacent to the surface of the body so that blood flowing through the body passes through a changing magnetic field. For example, EP 0 744 275-A2 discloses a magnotherapy device comprising a magnet having first and second magnetic poles, the magnet being associated with a flat shield or keeper formed of ferrous material in contact with a second pole of the magnet. DE-U-94 13 430 discloses a magnotherapy device having a magnetic core enclosed within a metal housing with a ring magnet cemented to the outside of the housing. A plastic component completely separates the magnetic core from the housing.

The present invention seeks to provide a new and inventive form of magnotherapy device.

### SUMMARY OF THE INVENTION

The present invention proposes a magnotherapy device having the characterising features of Claim 1.

The width of the gap between the side wall and the magnet is preferably between 0.1mm and 5mm, preferably less than 2mm. If the gap is small or non-existent the field strength adjacent to the first pole becomes too small, but on the other hand, as the size of the gap increases the flux concentration decreases.

### BRIEF DESCRIPTION OF THE DRAWINGS

The following description and the accompanying drawings referred to therein are included by way of non-limiting example in order to illustrate how the invention may be put into practice. In the drawings:

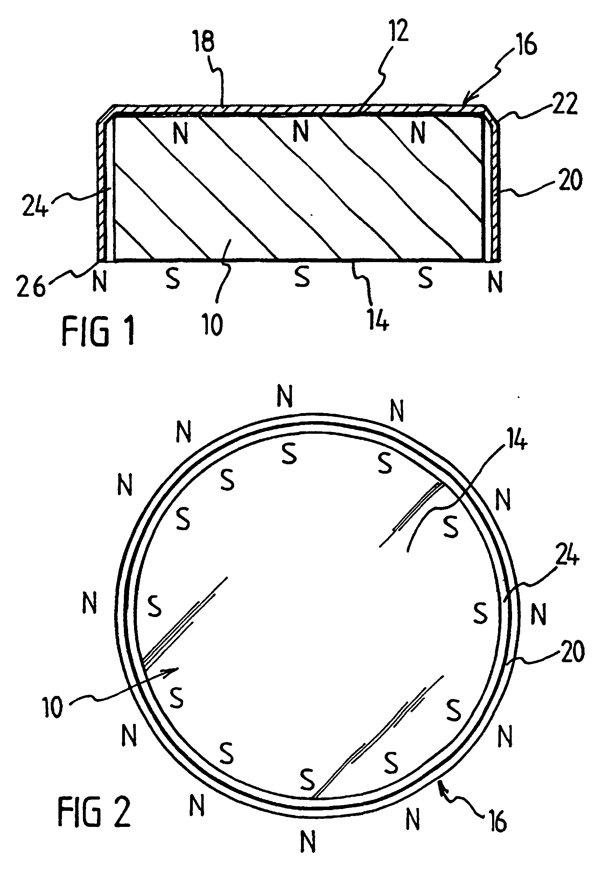

Figure 1 is an axial section through a magnotherapy device in accordance with the invention,
Figure 2 is a plan view of the end face of the device,
Figure 3 is a general view of a housing containing a modified form of the device, and
Figure 4 is a transverse section through the device and housing shown in Fig. 3.

### DETAILED DESCRIPTION OF THE DRAWINGS

The magnotherapy device comprises a short cylindrical magnet 10 having opposite north and south poles (N and S) disposed at its flat end faces 12 and 14 respectively. The magnet is housed within a cup-shaped ferrous metal shield 16, which includes a circular base wall 18 surrounded by a cylindrical side wall 20. The shield is formed by a pressing operation which causes a small radius or chamfer 22 to be formed at the junction between the base wall 18 and the side wall 20, but this is exaggerated in the drawing for the purpose of illustration. The outer diameter of the magnet 10 is less than the minimum diameter of the chamfer 22, and the opposed faces of the magnet and base wall 18 are true and flat so that magnetic attraction causes the end face 12 of the magnet to be pulled strongly against the base wall 18 resulting in a self-centring action due to the radius or chamfer 22. As a result, a substantially uniform gap 24 is formed between the magnet 10 and the side wall 20. The size of the gap will normally be between 0.1mm and 2mm.

Although the magnet may be held in the shield by an adhesive, even a thin layer of adhesive would introduce a further gap which would reduce the effectiveness of the device. It is therefore preferred to hold the magnet in the shield by magnetic attraction.

The free end 26 of the side wall 20 is substantially coplanar with the end face 14 of the magnet 10. The lines of magnetic flux from the end face 12 are directed through the shield forming a north magnetic pole which substantially surrounds the exposed south pole face 14. A highly concentrated field strength is thus created in this region.

As will shortly be described, the device will normally be mounted within a plastic or other non-ferrous housing provided with means for securing the housing to the body of a human or an animal with a contact surface of the housing positioned against the body. The planar end face 14 is either exposed and substantially flush with this contact surface or is separated therefrom by a thin non-ferrous wall. Thus, blood flowing in a direction which is substantially parallel to the contact surface is successively subjected to two changes in the magnetic field, N-S-N. It will be appreciated that such a change in field polarity will occur irrespective of the radial direction in which the blood flows across the face 14, so that the magnet assembly is substantially more effective than known forms of magnet.

If the magnet is inserted into the shield 20 in an inverted position the poles will be reversed, but the blood will be again be subjected to two changes in the magnetic field, S-N-S, irrespective of the radial direction of flow.

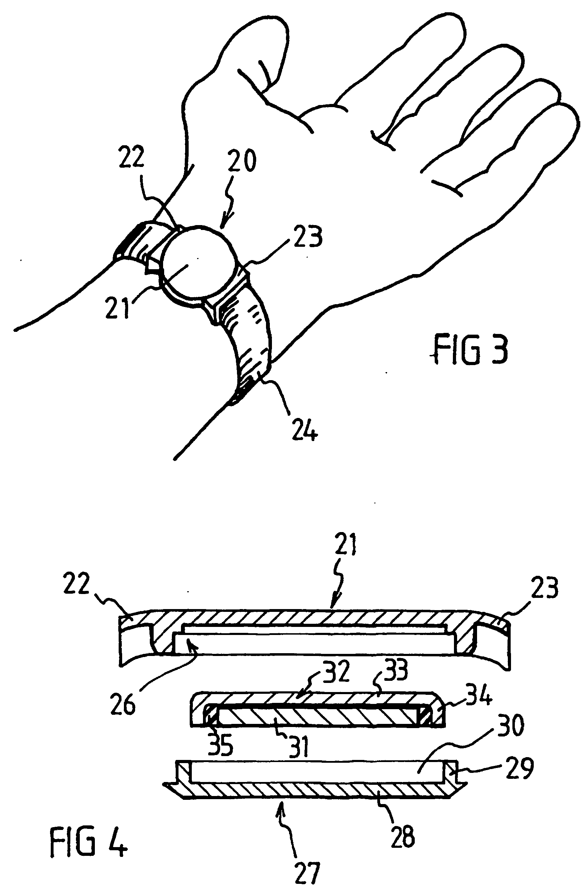

In Fig. 3 a modified form of the device is contained within a moulded plastics housing 20. The housing comprises a hollow circular part 21 with opposed projections 22 and 23 to which the ends of a wrist band 24 are secured. The wrist band may be of the kind formed of a continuous expandable loop or it may be in two sections which are mutually securable by a clasp or similar device.

As can be seen in the sectional view of Fig. 4, the hollow part 21 of the housing comprises a shallow container 26 and a closure or back 27. The back 27 includes a round flat wall 28 and a shallow cylindrical wall 29 forming a shallow circular well 30. The outer face of the wall 29 is shaped to snap-engage in the container portion 26, although the two parts of the housing could be secured together by other means, e.g. by an adhesive or by welding. A round magnet 31 having opposite north and south poles disposed at its flat end faces is housed within a cup-shaped ferrous metal shield 32. The shield includes a circular base wall 33 surrounded by a cylindrical side wall 34 and is formed by a pressing operation. The opposed faces of the magnet and base wall 33 are true and flat to form a strong magnetic connection. The free end of the side wall 34 is substantially coplanar with the end face of the magnet 31, and an annular space formed between the magnet and the side wall 34 is substantially filled by a tubular spacer formed by an annulus 35 of polythene or similar non-ferrous plastics material. In this case the size of the gap is about 1mm.

The magnet 31 and shield 32 are located in the well 30 of back part 27 with the exposed magnetic pole located against the wall 28. Thus, when the back 27 is engaged in to container part 26 the magnet and shield are held securely within the housing.

The lines of magnetic flux from the end face are directed through the shield forming a magnetic pole which substantially surrounds the exposed opposite pole face, creating a highly concentrated field strength at the periphery of the magnet, which extends through the back 27. Thus when the housing is worn with the back 27 placed against the body, blood flowing in a direction which is substantially parallel to the contact surface is successively subjected to two changes in the magnetic field irrespective of the radial direction in which the blood flows across the face.

It will be appreciated that the features disclosed herein may be present in any feasible combination.

## Claims

1. A magnotherapy device comprising a magnet (10, 31) having first and second magnetic poles, the magnet being enclosed within a metal housing (16, 32) including a base wall (18, 33) and a side wall (20, 34) which substantially surrounds the magnet but is spaced therefrom with a substantially uniform separation (24, 35) between the magnet and the side wall, and the free edge of the side wall is substantially coplanar with an end face of the magnet,
***characterised in that*** the housing is formed of a ferrous metal providing a magnetic shield (16, 32) around the magnet, the first pole of the magnet is disposed at said end face and the second pole of the magnet is in contact with the base wall of the shield such that lines of magnetic flux from the second pole are directed through the shield to form an opposite magnetic pole surrounding said first pole.

2. A magnotherapy device according to Claim 1, in which the device has no additional magnets outside the shield.

3. A magnotherapy device according to Claim 1 or 2, in which the distance between the side wall and the magnet is between 0.1mm and 2mm.

4. A magnotherapy device according to any preceding claim, in which there is a gap between the side wall and the magnet which is substantially filled with air.

5. A magnotherapy device according to any of Claims 1 to 3, in which the side wall and the magnet are separated by a plastics spacer.

6. A magnotherapy device according to any preceding claim, in which the magnet is substantially cylindrical.

7. A magnotherapy device according to any preceding claim, in which the magnet (10, 31) is held in the shield (16, 32) by magnetic attraction.

8. A magnotherapy device according to any preceding claim, in which the magnet and shield are mounted in a non-ferrous housing (20) provided with means (24) for securing the housing onto the body with a contact surface of the housing positioned against the body.

9. A magnotherapy device according to Claim 8, in which the housing comprises two parts:
- a container (26) to which said fastening means is secured, and
- a closure (27) for engagement with said container.

10. A magnotherapy device according to Claim 9, in which the closure is formed with a well (30) for receiving and locating said magnet and shield.

## Patentansprüche

1. Magnettherapiegerät mit einem Magneten (10, 31) mit ersten und zweiten magnetischen Polen, wobei der Magnet von einem Metallgehäuse (16, 32) umschlossen ist, das eine Bodenwand (18, 33) und eine Seitenwand (20, 34) aufweist, die den Magneten im Wesentlichen umfasst, aber auf Abstand mit einem im Wesentlichen gleichmäßigen Abstand 824, 35) zwischen dem Magneten und der Seitenwand liegt, und wobei die freie Endkante der Seitenwand im Wesentlichen koplanar mit einer Endfläche des Magneten ist,
**dadurch gekennzeichnet, dass** das Gehäuse aus einem eisenhaltigen Metall gebildet ist, das eine magnetische Abschirmung (16, 32) um den Magneten schafft, wobei der erste Pol des Magneten an der Endfläche und der zweite Pol des Magneten in Kontakt mit der Bodenwand der Abschirmung angeordnet ist, so dass die magnetischen Flusslinien von dem zweiten Pol durch die Abschirmung geleitet werden, um einen den ersten Pol umgebenden entgegengesetzten magnetischen Pol zu bilden.

2. Magnettherapiegerät nach Anspruch 1, bei dem das Gerät keine zusätzlichen Magneten außerhalb der Abschirmung hat.

3. Magnettherapiegerät nach Anspruch 1 oder 2, bei dem der Abstand zwischen der Seitenwand und dem Magneten zwischen 0,1 mm und 2 mm beträgt.

4. Magnettherapiegerät nach einem der vorhergehenden Ansprüche, bei dem eine Lücke zwischen der Seitenwand und dem Magneten vorhanden ist, die im Wesentlichen mit Luft gefüllt ist.

5. Magnettherapiegerät nach einem der Ansprüche 1 bis 3, bei dem die Seitenwand und der Magnet durch einen Kunststoffabstandshalter getrennt sind.

6. Magnettherapiegerät nach einem der vorhergehenden Ansprüche, bei dem der Magnet im Wesentlichen zylindrisch ist.

7. Magnettherapiegerät nach einem der vorhergehenden Ansprüche, bei dem der Magnet (10, 31) in der Abschirmung (16, 32) durch magnetische Anziehung festgehalten wird.

8. Magnettherapiegerät nach einem der vorhergehenden Ansprüche, bei dem der Magnet und die Abschirmung in einem nicht eisenhaltigen Gehäuse (20) angebracht sind, das mit Einrichtungen (24) zum Befestigen des Gehäuses auf dem Körper versehen ist, wobei eine Kontaktoberfläche des Gehäuses am Körper platziert ist.

9. Magnettherapiegerät nach Anspruch 8, bei dem das Gehäuse zwei Teile aufweist:
einen Behälter (26), an dem die Befestigungseinrichtungen angebracht sind, und
einen Verschluss (27) zum Eingriff mit dem Behälter.

10. Magnettherapiegerät nach Anspruch 9, bei dem der Verschluss mit einer Vertiefung (30) zur Aufnahme und Positionierung des Magneten und der Abschirmung ausgebildet ist.

## Revendications

1. Dispositif de magnétothérapie comprenant un aimant (10, 31) ayant des premier et second pôles magnétiques, l'aimant étant enfermé à l'intérieur d'un boîtier métallique (16, 32) comprenant une paroi de base (18, 33) et une paroi latérale (20, 34) qui entoure l'aimant de façon substantielle mais est espacée de celui-ci avec une séparation sensiblement uniforme (24, 35) entre l'aimant et la paroi latérale, et la bordure libre de la paroi latérale étant sensiblement coplanaire avec une face d'extrémité de l'aimant,
***caractérisé par le fait que*** le boîtier est formé d'un métal ferreux fournissant un écran magnétique (16, 32) autour de l'aimant, le premier pôle de l'aimant est disposé au niveau de ladite face d'extrémité et le second pôle de l'aimant est en contact avec la paroi de base de l'écran, de telle sorte que des lignes de flux magnétique provenant du second pôle sont dirigées à travers l'écran afin de former un pôle magnétique opposé entourant ledit premier pôle.

2. Dispositif de magnétothérapie selon la revendication 1, dans lequel le dispositif n'a pas d'aimants supplémentaires à l'extérieur de l'écran.

3. Dispositif de magnétothérapie selon l'une des revendications 1 ou 2, dans lequel la distance entre la paroi latérale et l'aimant est entre 0,1 mm et 2 mm.

4. Dispositif de magnétothérapie selon l'une quelconque des revendications précédentes, dans lequel il y a un intervalle entre la paroi latérale et l'aimant qui est sensiblement rempli d'air.

5. Dispositif de magnétothérapie selon l'une quelconque des revendications 1 à 3, dans lequel la paroi latérale et l'aimant sont séparés par un espaceur en matière plastique.

6. Dispositif de magnétothérapie selon l'une quelconque des revendications précédentes, dans lequel l'aimant est sensiblement cylindrique.

7. Dispositif de magnétothérapie selon l'une quelconque des revendications précédentes, dans lequel l'aimant (10, 31) est maintenu dans l'écran (16, 32) par attraction magnétique.

8. Dispositif de magnétothérapie selon l'une quelconque des revendications précédentes, dans lequel l'aimant et l'écran sont montés dans un boîtier non-ferreux (20) doté d'un moyen (24) pour fixer le boîtier sur le corps avec une surface de contact du boîtier positionnée contre le corps.

9. Dispositif de magnétothérapie selon la revendication 8, dans lequel le boîtier comprend deux parties :
- un conteneur (26) auquel ledit moyen de fixation est fixé ; et
- une fermeture (27) en vue d'un engagement avec ledit conteneur.

10. Dispositif de magnétothérapie selon la revendication 9, dans lequel la fermeture est formée avec un puits (30) pour recevoir et positionner ledit aimant et ledit écran.
